# EUROPÄISCHE PATENTSCHRIFT

(11) **EP 2 388 037 B1**
(45) Veröffentlichungstag und Bekanntmachung des Hinweises auf die Patenterteilung: **27.07.2016**
(21) Anmeldenummer: 11006459.9
(22) Anmeldetag: 01.09.2005
(51) Int. Cl.: A61M 16/06

(54) **Atemmaske**
Respiratory mask
Masque respiratoire

(30) Priorität: 03.09.2004 DE 102004043208
(43) Veröffentlichungstag der Anmeldung: 23.11.2011
(62) Teilanmeldung aus: 05787298.8
(73) Patentinhaber: Weinmann Geräte für Medizin GmbH + Co. KG, 22525 Hamburg (DE)
(72) Erfinder: Schulz, Gerd, 22869 Schenefeld (DE); Eifler, Martin, 25348 Glückstadt (DE)
(74) Vertreter: Patentanwälte Klickow & Partner Partnerschaftsgesellschaft mbB

(56) Entgegenhaltungen:
- EP-A2- 1 314 446
- WO-A1-2004/022145
- US-A- 5 921 239
- US-A1- 2003 075 180

## Beschreibung

Die Erfindung betrifft eine Atemmaske mit einem Maskenkörper und einem gelenkigen Anschlussstück, welches mit einem Atemschlauch verbindbar ist, wobei zur Bildung mindestens eines Ausatemspaltes zwischen zwei Ausströmflächen die Ausströmflächen durch eine Verspannung weitgehend toleranzfrei aneinandergedrückt sind und wobei eine Spalthöhe durch Distanzelemente zwischen den Ausströmflächen eingestellt ist.

Atemmasken werden beispielsweise im Zusammenhang mit Beatmungsgeräten verwendet, um Atemgas zum Patienten zu leiten und eine Ableitung des ausgeatmeten Atemgases zu unterstützen. Die Atemmaske ist typischerweise über den Atemschlauch mit dem Beatmungsgerät verbunden.

Als Nachteil bei den bereits bekannten Atemmasken erweist es sich, dass beim Ausatmen durch den Maskenkörper und den Beatmungsschlauch mit einer für den Patienten und die Umgebung unangenehmen akustischen Beeinträchtigung zu rechnen ist und darüber hinaus der ausgeatmete Luftstrom einen am Patienten entlang streichenden kühlen Luftzug hervorruft.

In der US 5921239 A wird eine Atemmaske mit einem Maskenkörper beschrieben, der ein gelenkiges Anschlussstück aufweist. Das Anschlussstück ist mit einem Atemschlauch verbindbar. Zwei Bauteile der Atemmaske können miteinander verbunden werden.

In der US 2003/075180 A1 wird ebenfalls eine Atemmaske mit einem Maskenkörper und einem gelenkigen Anschlussstück beschrieben. Auch hier ist das Anschlussstück mit einem Atemschlauch verbindbar und die Atemmaske besitzt mindestens zwei miteinander verbundene Bauteile.

Eine weitere Atemmaske mit Anschlussmöglichkeit an einen Atemgasschlauch wird in der WO 2004/022145 A1 erläutert.

Eine weitere Atemmaske ist aus der EP 1314446 A2 bekannt. Es ist hier ein Ausatemspalt zwischen einem Halterungsstutzen und einem Ausatmungselement angeordnet.

Aufgabe der vorliegenden Erfindung ist es, eine komfortable Gestaltung einer Atemmaske bereitzustellen, bei der die Beeinträchtigung des Patienten durch ausgeatmete Luft weitgehend vermieden wird.

Diese Aufgabe wird erfindungsgemäß dadurch gelöst, dass die eine Ausströmfläche im Maskenkörper und die komplementäre andere Ausströmfläche an einem Sicherungsring mit einer Schließeinrichtung ausgebildet ist, mittels dem eine Fixierung vom Maskenkörper und Anschlussstück zueinander erfolgt.

Gemäß einer weiteren Ausführungsform ist insbesondere in Kombination mit einer Atemmaske mit einem Maskenkörper und einem gelenkigen Anschlussstück, welches mit einem Atemschlauch verbindbar ist, auch daran gedacht, dass im Bereich des Maskenkörpers, bevorzugt im Bereich eines das gelenkige Anschlussstück aufnehmenden Anschlusses am Maskenkörper, mindestens ein Ausatemspalt angeordnet ist.

Jedes der in den Unteransprüchen definierten Merkmale und jedes Einzelmerkmal gemäß den Ausführungsbeispielen in der nachfolgenden Beschreibung kann einzeln oder in Kombination miteinander auch mit der Ausführungsform gemäß der im vorangegangenen Absatz definierten Konstruktion verwendet werden.

Die erfindungsgemäße Atemmaske, die nicht nur als Einzelteil sondern auch als Element eines ganzen Beatmungsgeräts zu verstehen ist, umfaßt einen Maskenkörper sowie ein gelenkiges Anschlussstück, welches mit einem Atemschlauch verbindbar ist, wobei im Bereich des Übergangs zwischen dem gelenkigen Anschlussstück und einem das gelenkige Anschlussstück aufnehmenden Anschluss am Maskenkörper mindestens ein Ausatemspalt angeordnet ist. Insbesondere die Anordnung des Ausatemspalts zwischen dem Maskenkörper und dem Anschlussstück hält den Geräuschpegel gegenüber anderen Anordnungspositionen gering. Auch die CO₂ - Auswaschung wird in dieser Position des Ausatemspalts besonders effektiv durchgeführt. Des Weiteren ist es gerade im Übergangsbereich zwischen Maskenkörper und Anschlußstück möglich, eine große Spaltlänge vorzusehen, die schalltechnisch besonders vorteilhaft ist im Vergleich beispielsweise zu Löchern und kurzen Schlitzen.

Der mindestens eine Ausatemspalt wird bevorzugt von zwei Ausströmflächen begrenzt.

Das gelenkige Anschlußstück ist vorteilhafterweise als Kugelgelenk ausgebildet und liegt an einzelnen Punkten, insbesondere an zwei Punkten, in einem Kugelkäfig des aufnehmenden Anschlusses auf. Auf diese Weise tritt eine geringe Lagerreibung auf und eine leichte Beweglichkeit des angeschlossenen Atemschlauchs ist gewährleistet. Ebenfalls können in einfacher Weise Toleranzen ausgeglichen werden.

In einer weiteren vorteilhaften Ausgestaltung der Atemmaske wird der mindestens eine Ausatemspalt von den Ausströmflächen begrenzt, wobei diese sich benachbart zu mindestens einem Distanzelement befinden und dabei spielfrei oder sogar mit Vorspannung zueinander versehen sind. So wird vermieden, dass durch entweichende Luft bei bestehendem Spiel unerwünschte Schwingungen und Resonanzen auftreten. Insbesondere bei einem Ausatemspalt in einem Kugelgelenk ergibt sich nämlich eine große Spaltlänge. Dies könnte sich unter Umständen als schalltechnisch nachteilhaft erweisen, wenn sich im Spalt bewegliche Teile befinden.

Der Ausatemspalt zwischen zwei Ausströmflächen kann zudem weitgehend toleranzfrei hergestellt werden, wenn die spaltbildenden Flächen durch eine Vorspannung aneinandergedrückt werden und hierdurch kein Spiel aufweisen, und die Spalthöhe durch Distanzelemente zwischen den Flächen eingestellt wird. Bei einer solchen Konstruktion hängt die Spalthöhe abgesehen von Form- und Lagetoleranzen der spaltbildenden Flächen nur von der Toleranz einer Rippenhöhe als Distanzelement ab. Da typische Rippenhöhen in Ausatemspalten zwischen 0,1 und 0,5 mm liegen, sind Spalttoleranzen von +-0,005 mm und feiner prozeßsicher herstellbar. Flow- und Schallemissionen werden folglich in sehr engen Grenzen gehalten.

Die Verbindung von Maskenkörper und Anschlußstück kann bevorzugt mit einer mechanischen Kodierung erfolgen. Das hat zum Vorteil, dass eine fehlerhafte Fixierung von Maskenkörper und Anschlußstück zueinander sowie eine fehlerhafte Kombination eines Anschlußstücks mit einem Maskenkörper verhindert werden.

Die Fixierung von Maskenkörper und Anschlußstück zueinander erfolgt vorteilhaft über einen Sicherungsring mit einer Schließeinrichtung, beispielsweise mit einem Bajonettverschluß. Durch den Sicherungsring werden auch die Ausatemspalte fixiert, indem zum Beispiel die Ausströmflächen der Atemmaske und komplementäre Ausströmflächen am Sicherungsring gegeneinander vorgespannt werden.

Eine zusätzliche vorteilhafte Ausgestaltung der Atemmaske besteht darin, dass die Ausströmflächen einen Ausströmkanal bilden, der der Atemgasstrom in einem Winkel zwischen 10° und 45°, insbesondere zwischen 20° und 30°, besonders bevorzugt von etwa 25°, bezogen auf eine Ebene frontal zum Gesicht eines Patienten, ableitet. Auf diese Weise wird die ausgeatmete Luft im Wesentlichen schirmförmig vom Patienten weggeleitet und macht sich für den Patienten nicht unangenehm bemerkbar.

Des Weiteren kann der Abströmkanal so ausgebildet sein, dass er ein Ableiten der Ausatemströmung in Richtung der Augenpartie des Patienten ausschließt, da sich ein Luftzug an den Augen als besonders unangenehm erweist.

Weiterhin kann die Atemmaske so ausgebildet sein, dass an den Ausströmungskanal grenzende Oberflächen der Atemmaske aus hartem Kunststoff gefertigt sind und Oberflächen von mit der Hand bedienbaren Elementen der Atemmaske aus weichem Kunststoff gefertigt sind. Auf diese weise wird einerseits der Atemstrom zuverlässig abgeleitet und andererseits die Handhabbarkeit der Atemmaske erleichtert.

Die Erfindung wird im Folgenden beispielhaft anhand der Figuren erläutert. Es zeigen:
- Fig. 1: eine als Nasalmaske ausgebildete Atemmaske in perspektivischer Ansicht,
- Fig. 2: eine Ansicht auf das Innere der Maske aus Fig. 1 von hinten,
- Fig. 3: eine Ansicht auf das Äußere der Maske aus Fig. 1 von vorne,
- Fig. 4: den Grundkörper der Maske aus Fig. 1 in perspektivischer Ansicht,
- Fig. 5: eine Seitenansicht des Grundkörpers,
- Fig. 6: einen Querschnitt durch die Symmetrieebene des Grundkörpers,
- Fig. 7: eine Ansicht auf den Sicherungsring von hinten,
- Fig. 8: eine Ansicht auf den Sicherungsring von vorne,
- Fig. 9: den Sicherungsring in perspektivischer Ansicht,
- Fig. 10: den Sicherungsring in seitlicher Ansicht,
- Fig. 11: den Sicherungsring aus Fig. 10 im Querschnitt,
- Fig. 12: eine perspektivische Darstellung des Maskengrundkörpers,
- Fig. 13: eine teilweise Darstellung eines vergrößerten Schnittes durch den Maskengrundkörper im Bereich des Kugelgelenkes und
- Fig. 14: eine vergrößerte Darstellung der Einzelheit XIV in Fig. 13.

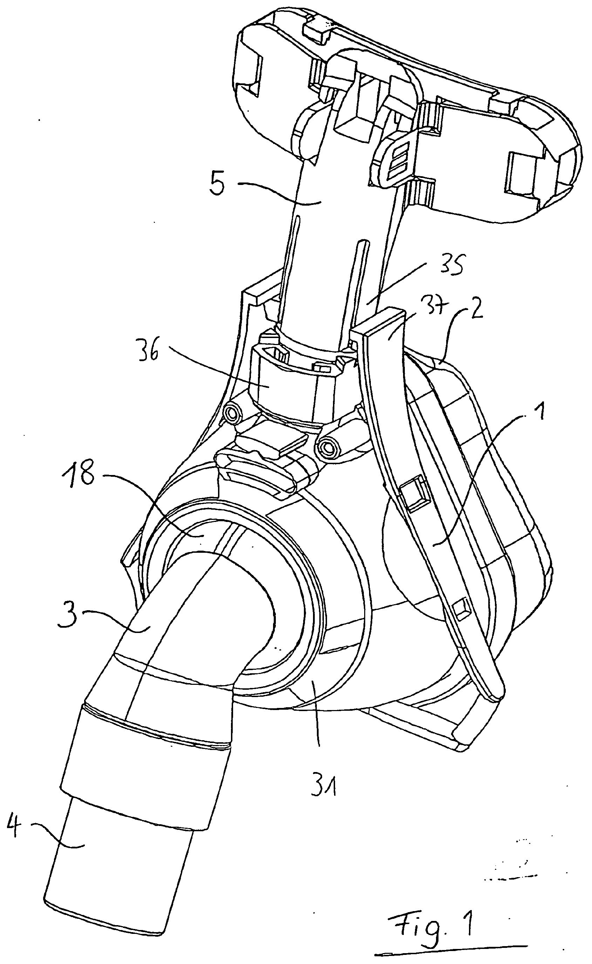

Fig. 1 zeigt eine als Nasalmaske ausgebildete Atemmaske, deren Maskenkörper (1) aus einem relativ festen Material gefertigt ist und die einen Maskenwulst (2) aufweist. Der Maskenwulst (2) dient zur Anlage am Gesicht eines nicht abgebildeten Patienten und gewährleistet die erforderliche Abdichtung. Über ein winkelförmig ausgebildetes Anschlußstück (3) ist der Maskenkörper (1) mit einer drehbeweglich gelagerten Hülse (4) verbunden, die zum Anschluß an einen nicht abgebildeten Atemgasschlauch dient. Zur Gewährleistung einer sicheren Positionierung der Atemmaske im Kopfbereich eines Patienten wird eine Stirnstütze (5) verwendet. Das Anschlußstück (3) und der Maskenkörper (1) sind über ein Kugelgelenk (18) miteinander verbunden.

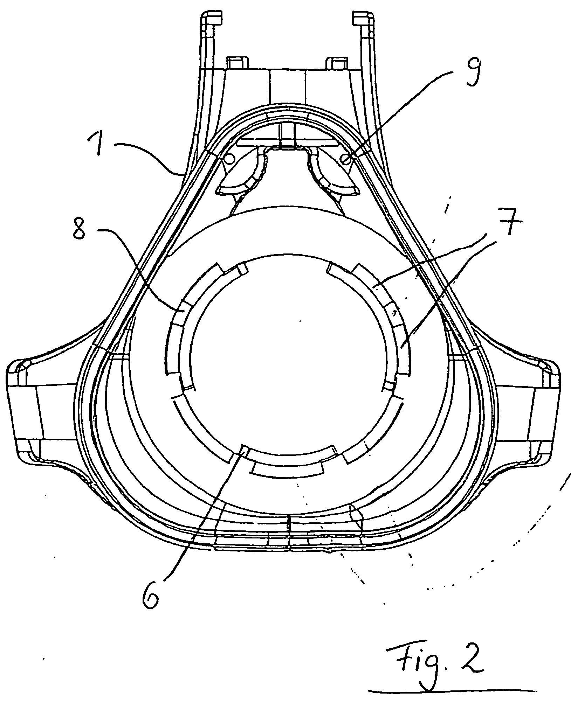

In Fig. 2 ist das Innere der Nasalmaske aus Fig. 1 bei einer Blickrichtung von innen in Richtung auf eine Aufnahme für das hier nicht abgebildete Kugelgelenk (18) dargestellt. Im oberen Bereich sind zwei Druckmeßstutzen (9) angeordnet. Strömungsöffnungen (7) führen zu nicht abgebildeten Ausatemspalten (14). Die Strömungsöffnungen (7) sind von Strömungsleitstrukturen (8) begrenzt. Die Strömungsleitstrukturen (8) sind derart ausgebildet, dass der Atemgasstrom gesammelt und in die Ausatemspalten (14) geleitet wird. Die Strömungsleitstrukturen (8) sind bevorzugt derart ausgebildet, dass der Atemgasstrom trichterförmig in die Ausatemspalten (14) geleitet wird. Die Strömungsleitstruktur (8) im oberen Bereich verschließt den Atemgasstrom in Richtung der Augen eines Anwenders. Einführschrägen (6) sind für das erleichterte Einführen von Bajonettzähnen (26) eines nicht dargestellten Kugelkäfigs (24) vorgesehen.

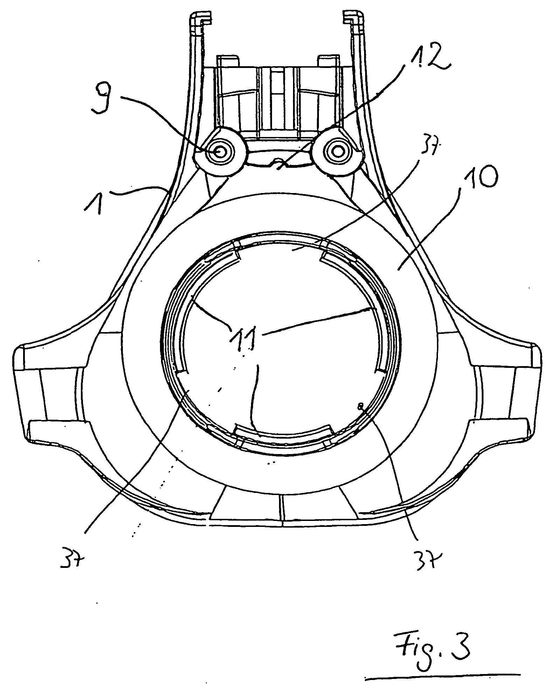

Fig. 3 gibt den Blick auf das Äußere der Maske aus Fig. 1 bei einer Blickrichtung von vorne frei. Eine Ausströmfläche (10) ist ringförmig ausgebildet. Mit Hilfe von Rippen (11) und einer Verrastung (12) kann ein hier nicht abgebildeter Sicherungsring (31) angebracht und fixiert werden.

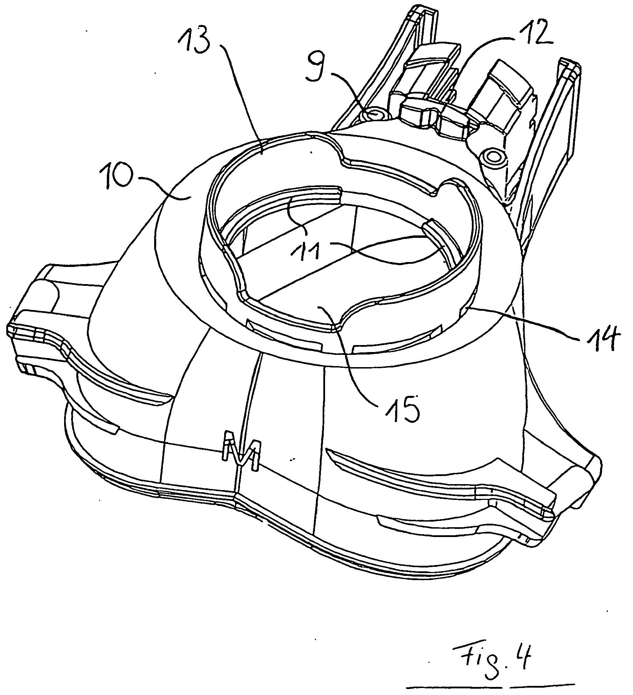

In Fig. 4 ist der Grundkörper der Nasalmaske in perspektivischer Ansicht dargestellt. Ein Zentrierring (13) ist für das Aufsetzen des Sicherungsrings vorgesehen, Ausatemspalte (14) befinden sich an den Seiten des Zentrierrings (13) und öffnen sich zur Ausströmfläche (10) hin. Der Zentrierring (13) weist Aussparungen (15) auf, die eine Fehlmontage des Sicherungsrings verhindern, indem sie eine mechanische Kodierung bilden.

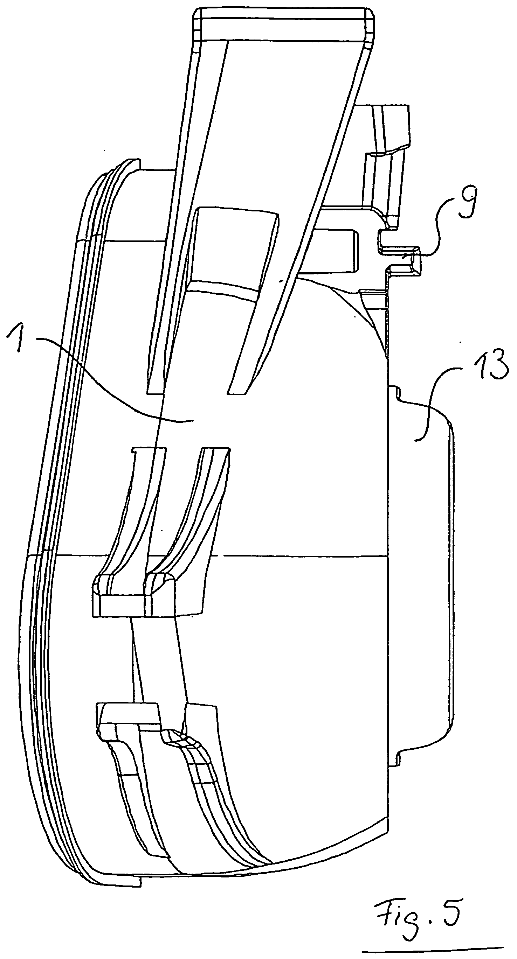

In Fig. 5 ist eine Seitenansicht des Grundkörpers dargestellt. Zu erkennen sind insbesondere der Maskenkörper (1), der Zentrierung (13) und die Verrastung (12).

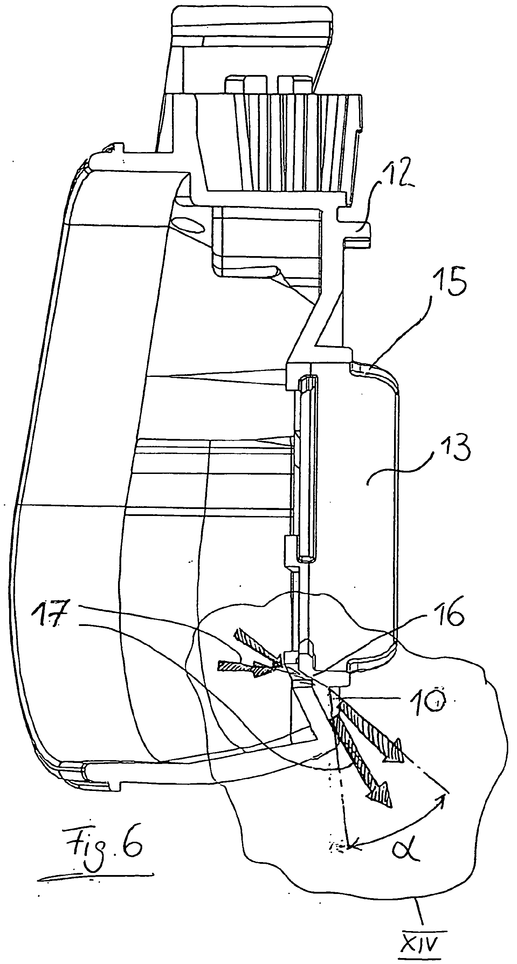

In der Fig. 6 ist der Grundkörper aus Fig. 5 im Querschnitt dargestellt. Der Atemgasstrom (17) gelangt entlang der Strömungsleitstruktur (8) in den Ausatemspalt (14). Die Strömungsleitstruktur (8) ist bevorzugt derart ausgebildet, dass der Atemgasstrom trichterförmig in den Ausatemspalt (14) geleitet wird. Der Ausatemspalt ist durch die Strömungsleitfläche (16) begrenzt. Im Ausatemspalt wird der Atemgasstrom zumindest einmal an der Strömungsleitfläche (16) umgelenkt. Es kann eine Umlenkung der Atemgasströmung nur im Ausatemspalt (14), nur im Bereich der Überleitung des Maskengrundkörpers (1) in den Ausatemspalt (14) oder kombiniert in beiden Bereichen erfolgen.

Entlang der Strömungsleitfläche (16) gelangt der Atemgasstrom vom Inneren der Atemmaske nach außen. Nach Verlassen des Ausatemspaltes (14), der die engste Stelle darstellt, verlässt der Atemgasstrom fächerförmig die Atemmaske entlang der langgestreckten Ausströmfläche (10). Dabei verläßt der Atemgasstrom den Ausatemspalt (14) in einem Winkel Alpha, der vorzugsweise zwischen 10° und 45° zur Vertikalen der Fig. 6 liegt. Diese vertikale Ebene fällt zusammen mit einer Ebene, die frontal zum Gesicht eines hier nicht dargestellten Patienten verläuft.

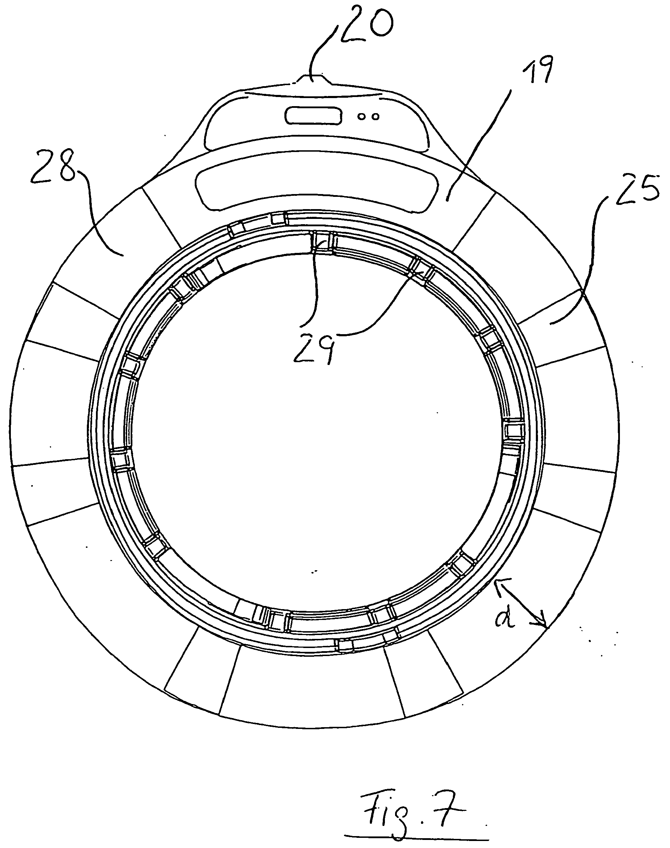

In Fig. 7 ist eine Draufsicht auf einen Sicherungsring (31) von hinten gezeigt, der eine Breite d von weniger als 7 mm aufweist, dargestellt durch einen Doppelpfeil. Distanzrippen (25) ermöglichen eine Montage des Sicherungsrings (31) auf dem Maskenkörper (1) im Bereich der Ausströmfläche (10) ohne Spiel bzw. mit Vorspannung. Ausströmflächen (28) am Sicherungsring (31) begrenzen im montierten Zustand mit der Ausströmfläche (10) am Maskenkörper (1) die Ausatemspalte (14). Eine Nase (20) am Sicherungsring (31) bildet das Komplement zur Verrastung (12) am Maskenkörper (1), so dass eine zusätzliche Befestigung des Sicherungsrings (31) am Maskenkörper (1) gewährleistet wird. Ein in montiertem Zustand an der Ausströmfläche (10) aufliegendes Flächensegment (19) gewährleistet, dass keine Luft in Richtung der Augen der Patienten abströmt. Schlitze (29) zwischen den Elementen des Kugelkäfigs (24) sorgen für eine leichte Montage.

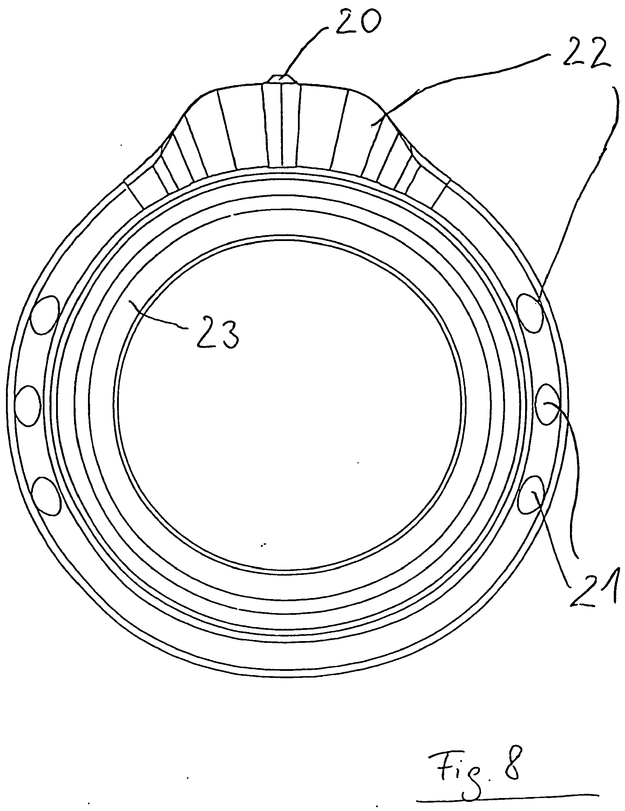

Die Fig. 8 zeigt eine Ansicht auf den Sicherungsring (31) von vorne. Der innere Bereich des Sicherungsrings (31) ist aus Hartmaterial (23) gefertigt, und der äußere Bereich aus einem Weichmaterial (22). Noppen (21) verbessern die Griffigkeit.

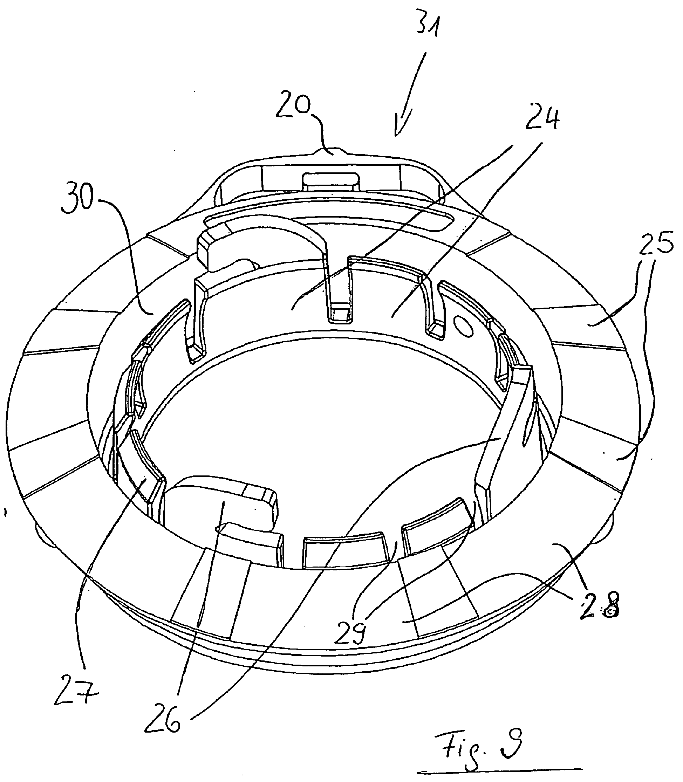

Die Fig. 9 stellt den Sicherungsring (31) in perspektivischer Ansicht von hinten dar. Diese Blickrichtung gewährleistet eine Sicht auf die Aufnahme (30) für den Steg des Zentrierrings (13).

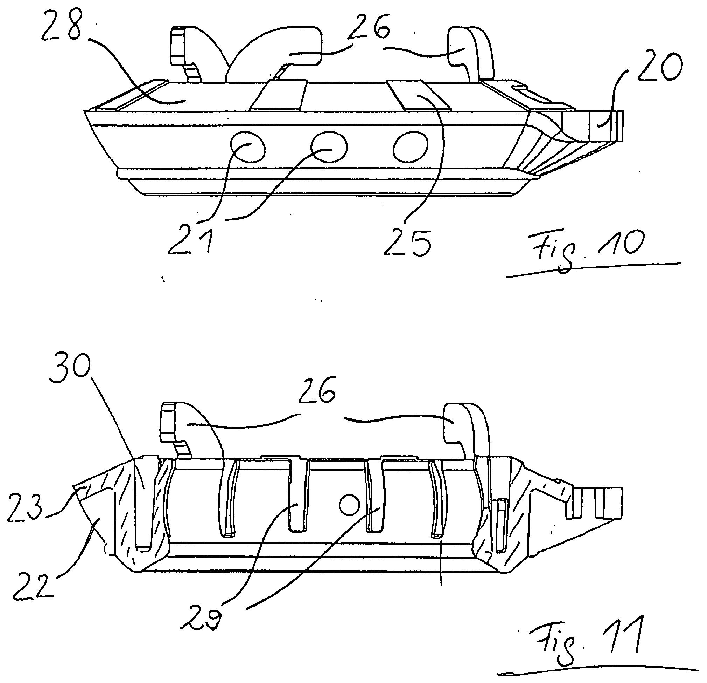

Die Fig. 10 zeigt den Sicherungsring (31) in seitlicher Ansicht und gewährt den Blick auf Bajonettzähne (26).

Die Fig. 11 gibt den Sicherungsring (31) aus Fig. 10 im Querschnitt wieder.

Die Funktionalität der einzelnen in Fig. 9 bis Fig. 11 dargestellten Bauelemente wird später nochmals ausführlich im Zusammenhang mit einer Beschreibung einer Montage und Demontage der einzelnen Bauelemente erläutert, um insbesondere die mechanische Bedeutung und Funktionalität der einzelnen Bauelemente weiter zu veranschaulichen.

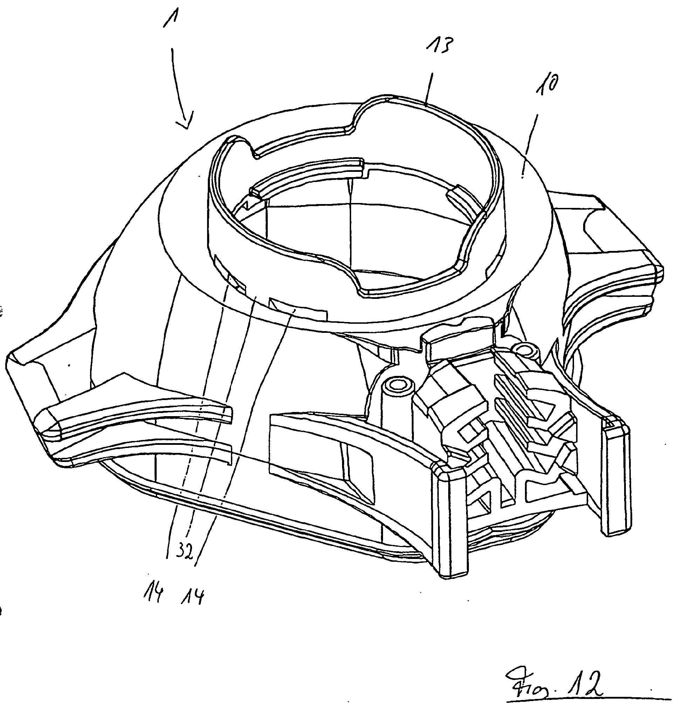

Die perspektivische Darstellung in Fig. 12 veranschaulicht nochmals den Maskenkörper (1) nach einem Abnehmen der in Fig. 12 nicht dargestellten Stirnstütze (5). Im Übergangsbereich zwischen der Ausströmfläche (10) und dem Zentrierring (13) ist insbesondere noch einmal die Anordnung der Ausatemspalte (14) zu erkennen. Die Ausatemspalte (14) besitzen im wesentlichen rechteckförmige Querschnittgestaltungen und verlaufen mit ihren Längsachsen in einer Umfangsrichtung der Ausströmfläche (10). Die einzelnen Ausatemspalte (14) sind von Distanzelementen (32) voneinander getrennt. Die Distanzelemente (32) führen zu einer mechanischen Verbindung zwischen dem Zentrierring (13) und dem weiteren Material des Maskenkörpers (1).

Die Ausatemspalte (14) sind vorzugsweise derart angeordnet, dass sie in einem der Ausströmfläche (10) zugewandten Bereich des Zentrierringes (13) verlaufen. Hierdurch wird das aus den Ausatemspalten (14) austretende Atemgas unmittelbar in den Bereich der Ausströmfläche (10) geleitet. Nach Verlassen der Ausatemspalten (14) wird der Atemgasstrom an den langgestreckten und breiten Ausströmflächen (10) (28) umgelenkt und strömt diffus und leise, durch den von den Ausströmflächen definierten Ausströmkanal, in die Umgebung ab.

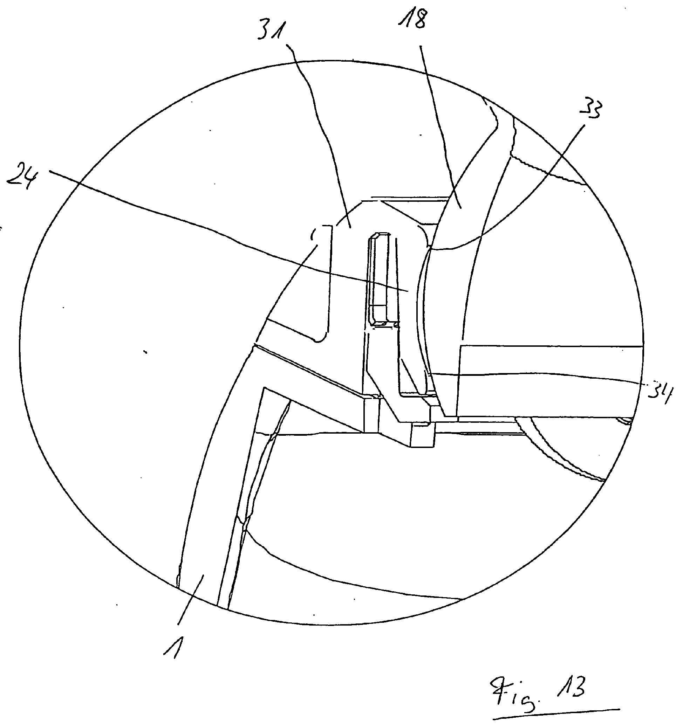

Fig. 13 zeigt eine vergrößerte teilweise Schnittdarstellung im Übergangsbereich zwischen dem Kugelgelenk (18) und dem vom Maskenkörper (1) gehalterten Sicherungsring (31). Zu erkennen ist insbesondere ein Wölbungsverlauf des Kugelkäfigs (24). Der Kugelkäfig (24) verläuft hierbei konkav mit einer stärkeren Krümmung als eine konvex ausgebildete äußere Oberfläche des Kugelgelenkes (18). Das Kugelgelenk (18) wird hierdurch nur entlang zweier Führungslinien, Führungspunkte (33, 34) oder entlang von Liniensegmenten vom Kugelkäfig (24) beaufschlagt. Hierdurch können eventuell im Bereich der Oberflächen des Kugelgelenkes (18) und/oder des Kugelkäfigs (24) vorliegende Fertigungstoleranzen ausgeglichen werden, da keine flächige Führung der Bauteile aneinander vorgesehen ist. Vorzugsweise erfolgt eine federnde Verspannung des Kugelgelenkes (18) innerhalb des Kugelkäfigs (24), damit ein gesichertes Aufliegen entlang der Führungslinien (33, 34) sichergestellt ist.

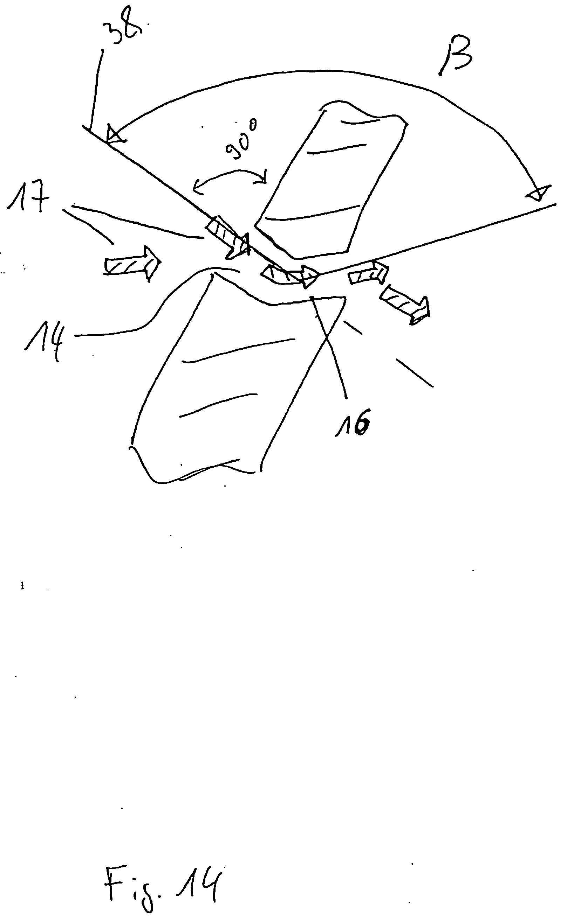

Fig. 14 zeigt einen vergrößerten Ausschnitt der Passage des Atemgasstromes durch den Ausatemspalt (14) entlang der Strömungsleitfläche (16) in die Umgebung (siehe Fig. 6).

Der Atemgasstrom (17) gelangt beispielsweise trichterförmig in den Ausatemspalt (14). Der Ausatemspalt ist durch die Strömungsleitfläche (16) begrenzt. Im Ausatemspalt wird der Atemgasstrom zumindest einmal an der Strömungsleitfläche (16) umgelenkt. Entlang der Strömungsleitfläche (16) gelangt der Atemgasstrom vom Inneren der Atemmaske nach außen. Bei der Passage des Atemgasstromes durch den Ausatemspalt (14) entlang der Strömungsleitfläche (16) in die Umgebung wird der Atemgasstrom in einem Winkel Beta (β), der vorzugsweise zwischen 1° und 15° zu einer Vertikalen (38) liegt abgelenkt. Diese Vertikale steht im wesentlichen in einem Winkel von 90° senkrecht zu der Ebene, die durch die den Ausatemspalt begrenzenden Teile des Maskenkörpers gebildet wird. Bei der Passage des Atemgasstromes durch den Ausatemspalt (14) entlang der Strömungsleitfläche (16) in die Umgebung wird der Atemgasstrom besonders bevorzugt in einem Winkel Beta (β), der zwischen 2° und 7° zu einer Vertikalen (38) liegt abgelenkt.

Zur weiteren Erläuterung der Funktionalität der einzelnen Bauelemente wird nunmehr ausgehend von einem demontierten Zustand die auch von einem Patienten in einfacher Weise vornehmbare Montage der Einzelteile erläutert. In einem ersten Schritt werden der aus dem härteren Material bestehende Maskenkörper (1) und der Maskenwulst (2) zusammengefügt. Der Maskenwulst (2) wird hierzu mit einem in den Zeichnungen nicht näher dargestellten U-Profil auf einen Rand des Maskenkörpers (1) aufgeschoben. Im Bereich der Kontaktstelle zwischen Maskenwulst und Maskenkörper befindet sich zumindest ein Hinterschnitt und zumindest ein zum Hinterschnitt komplementärer Vorsprung. Bevorzugt befindet sich der Hinterschnitt im Bereich des weicheren Bauteiles. Das Zusammenwirken von Hinterschnitt und Vorsprung im montierten Zustand sorgt für eine hohe Verbindungsfestigkeit.

In einem nächsten Montageschritt kann beispielsweise die Stirnstütze (2) mit einem Schaft (35) in eine Halterung (36) des Maskenkörpers (1) eingesetzt und unter Verwendung zumindest eines Befestigungsmittels (37) arretiert werden. Die Montage der Stirnstütze (5) kann aber auch zu beliebigen anderen Zeitpunkten des Montagevorganges erfolgen.

In einem weiteren Montageschritt wird der Sicherungsring (31) ausgehend von der Hülse (4) auf das Anschlußstück (3) aufgeschoben und im Bereich des Kugelgelenkes (18) positioniert. Das Aufschieben des Sicherungsringes (31) erfolgt hierbei in einer Orientierung derart, dass nach einem Abschluss des Aufsetzvorganges die Bajonettzähne (26) in eine der Hülse (4) abgewandte Richtung weisen und das Kugelgelenk (18) vom Kugelkäfig (24) des Sicherungsringes (31) bereichsweise umschlossen ist.

In einem abschließenden Montageschritt wird der Sicherungsring (31) gemeinsam mit dem Anschlußstück (3) im Bereich des Zentrierringes (13) des Maskenkörpers (1) positioniert. Aufgrund der beispielsweise in Fig. 3 zu erkennenden entlang eines Umlaufweges unsymmetrischen Anordnung der Rippen (11) und einer hierzu korrespondierenden Anordnung der Bajonettzähne (26) lassen sich die Bajonettzähne (26) nur in einer einzigen vorgegebenen Positionierung in die Aussparungen (37) zwischen den Rippen (11) einführen. Hierdurch wird eine Codierung bereitgestellt.

Nach dem Einführen der Bajonettzähne (26) in die Aussparungen (37) zwischen den Rippen (11) erfolgt eine Verdrehung des Sicherungsringes (31) relativ zum Maskenkörper (1) derart, dass die Verrastung (12) wirksam wird. Die Verrastung (12) wird vorzugsweise als ein Vorsprung des Sicherungsringes (31) ausgebildet, der in eine korrespondierende Vertiefung des Maskenkörpers (1) eingreift. Grundsätzlich ist aber auch eine umgekehrte Ausbildung denkbar. Ein elastisches Einrasten der Verrastung (12) wird dadurch unterstützt, dass der Sicherungsring (31) aus einem relativ weichen Material ausgebildet ist, so dass der ebenfalls weiche Vorsprung des Sicherungsringes (31) in die Ausnehmung des Maskenkörpers (1) einführbar und auch wieder aus dieser herausdrehbar ist.

Nach einer den Montagevorgang abschließenden Verdrehung des Sicherungsringes (31) relativ zum Maskenkörper (1) ist der Montagevorgang abgeschlossen. Die Endposition des Sicherungsringes (31) ist durch einen seitlichen Anschlag der Bajonettzähne (26) an den Rippen (11) vorgegeben. Die Bajonettzähne (26) hintergreifen darüber hinaus die Rippen (11), so dass die Gesamtanordnung auch Zugbelastungen standhält. Eine Demontage der Atemmaske erfolgt in umgekehrter Reihenfolge wie vorstehend für die Montage beschrieben.

Die Konstruktion des Sicherungsringes (31) führt dazu, dass der Sicherungsring (31) spielfrei und gegebenenfalls unter vorspannung auf den Maskenkörper (1) aufgesetzt werden kann. Dies hat zur Folge, dass die weite des oder der Ausatemspalte (14) nur noch von der Toleranz der Höhe der Distanzrippen (25) abhängig ist. Die Realisierung des Ausatemspaltes (14) erfolgt hierdurch äußerst gleichmäßig und weitgehend toleranzunabhängig. Dies wiederum hat zur Folge, dass die Abströmung des Atemgases durch den Ausatemspalt (14) hindurch und die hierdurch hervorgerufenen Schallemissionen äußerst konstant realisiert werden.

Die Konstruktion des Sicherungsringes (31) ermöglicht, dass das Kugelgelenk (18) in den federnden Kugelkäfig (24) des Sicherungsringes (31) eingeführt werden kann und dass der Kugelkäfig (24) das Kugelgelenk zumindest bereichsweise umschließt. Die Fixierung des Kugelgelenks (18) im Kugelkäfig (24) erfolgt dadurch, dass der Zentrierring (13) in die im Sicherungsring befindliche Aufnahme (30) eingeführt wird. Dadurch werden die federnden Elemente des Kugelkäfigs (24) auf der einen Seite durch das Kugelgelenk und auf der gegenüberliegenden Seite durch den Zentrierring (13) begrenzt. Im montierten Zustand ist dadurch das Kugelgelenk im Bereich der Maske fixiert. Die Beweglichkeit des Kugelgelenkes einerseits und die Abdichtung gegenüber dem Atemgas im Bereich zwischen Kugelgelenk und Kugelkäfig wird durch die genaue Dimensionierung und enge Toleranzmaße bestimmt.

## Patentansprüche

1. Atemmaske mit einem Maskenkörper (1) und einem gelenkigen Anschlussstück (3, 18), welches mit einem Atemschlauch verbindbar ist, wobei zur Bildung mindestens eines Ausatemspaltes (14) zwischen zwei Ausströmflächen (10, 28) die Ausströmflächen (10, 28) durch eine Vorspannung weitgehend toleranzfrei aneinandergedrückt sind und wobei eine Spalthöhe durch Distanzelemente (25, 32) zwischen den Ausströmflächen (10, 28) eingestellt ist, **dadurch gekennzeichnet, dass** die eine Ausströmfläche (10) im Maskenkörper (1) und die komplementäre andere Ausströmfläche (28) an einem Sicherungsring (31) mit einer Schließeinrichtung (20, 26) ausgebildet ist, mittels dem eine Fixierung von Maskenkörper (1) und Anschlussstück (3, 18) zueinander erfolgt.

2. Atemmaske nach Anspruch 1, **dadurch gekennzeichnet, dass** die Toteranzfreiheit durch einen Kontakt von Bauteilen (1, 31) an höchstens drei Punkten realisiert ist.

3. Atemmaske nach Anspruch 1 oder 2, **dadurch gekennzeichnet, dass** die Toleranzfreiheit durch einen Kontakt der Bauteile (1, 31) entlang von zwei Führungslinien (33, 34) realisiert ist.

4. Atemmaske nach einem der vorhergehenden Ansprüche, **dadurch gekennzeichnet, dass** die Bauteile (1, 31) zum Bilden des mindestens einen Ausatemspaltes (14) elastisch gegeneinander verspannt sind..

5. Atemmaske nach einem der vorhergehenden Ansprüche, **dadurch gekennzeichnet, dass** der mindestens eine Ausatemspalt (14) im Bereich eines das gelenkige Anschlussstück (3, 18) aufnehmenden Anschlusses (13) am Maskenkörper (1) angeordnet ist.

6. Atemmaske nach Anspruch 5, **dadurch gekennzeichnet, dass** der Anschluss (13) ein Zentrierring zum Aufsetzen des Sicherungsrings (31) ist, und dass im Übergangsbereich zwischen der Ausströmfläche (10) und dem Zentrierring (13) der mindestens eine Ausatemspalt (14) angeordnet ist.

7. Atemmaske nach einem der vorhergehenden Ansprüche, **dadurch gekennzeichnet, dass** das gelenkige Anschlussstück (3, 18) als Kugelgelenk ausgebildet ist.

8. Atemmaske nach Anspruch 6 oder 6 und 7, **dadurch gekennzeichnet, dass** der Sicherungsring (31) gemeinsam mit dem Anschlussstück (3, 18) im Bereich des Zentrierrings (13) des Maskenkörpers (1) positioniert ist.

9. Atemmaske nach Anspruch 7 oder 8, **dadurch gekennzeichnet, dass** im Übergangsbereich zwischen dem Kugelgelenk (18) und dem Maskenkörper (1) gehalterten Sicherungsring (31) eine federnde Verspannung des Kugelgelenkes (18) innerhalb des Kugelkäfigs (24) erfolgt.

10. Atemmaske nach Anspruch 9, **dadurch gekennzeichnet, dass** die Fixierung des Kugelgelenkes (18) erfolgt im Kugelkäfig (24) durch den Zentrierring (13) erfolgt der zumindest bereichsweise außen im Kugelkäfig (24) anliegt.

## Claims

1. A respiratory mask having a mask body (1) and an articulated connection piece (3, 18) which can be connected to a breathing tube, wherein, to form at least one exhalation gap (14) between two outflow surfaces (10, 28), the outflow surfaces (10, 28) are pushed against one another through pre-tension in substantially tolerance-free manner and wherein a gap height is adjusted by spacer elements (25, 32) between the outflow surfaces (10, 28), **characterised in that** the one outflow surface (10) is formed in the mask body (1) and the complementary other outflow surface (28) is formed on a retaining ring (31) having a closing means (20, 26), which retaining ring fixes the mask body (1) and connection piece (3, 18) to one another.

2. A respiratory mask according to Claim 1, **characterised in that** the freedom from tolerances is realised through contact between components (1, 31) at a maximum of three points.

3. A respiratory mask according to Claim 1 or 2, **characterised in that** the freedom from tolerances is realised through contact between the components (1, 31) along two guide lines (33, 34).

4. A respiratory mask according to one of the preceding claims, **characterised in that** the components (1, 31) are tensioned resiliently against one another to form the at least one exhalation gap (14).

5. A respiratory mask according to one of the preceding claims, **characterised in that** the at least one exhalation gap (14) is arranged in the region of a connection (13) on the mask body (1), which receives the articulated connection piece (3, 18).

6. A respiratory mask according to Claim 5, **characterised in that** the connection (13) is a centring ring for the attachment of the retaining ring (31) and **in that** the at least one exhalation gap (14) is arranged in the transition region between the outflow surface (10) and the centring ring (13).

7. A respiratory mask according to one of the preceding claims, **characterised in that** the articulated connection piece (3, 18) is constructed as a ball and socket joint.

8. A respiratory mask according to Claim 6 or 6 and 7, **characterised in that** the retaining ring (31) is positioned together with the connection piece (3, 18) in the region of the centring ring (13) of the mask body (1).

9. A respiratory mask according to Claim 7 or 8, **characterised in that**, in the transition region between the ball and socket joint (18) and the mask body (1) mounted retaining ring (31), a resilient pretensioning of the ball and socket joint (18) takes place within the ball cage (24).

10. A respiratory mask according to Claim 9, **characterised in that** the fixing of the ball and socket joint (18) takes place in the ball cage (24) takes place by means of the centring ring (13) which, at least in certain areas, rests externally in the ball cage (24).

## Revendications

1. Masque respiratoire, avec un corps de masque (1) et une pièce de raccordement articulée (3, 18), qui peut être reliée à un tuyau respiratoire, sachant que pour la formation d'au moins une fente d'expiration (14) entre deux surfaces de sortie (10, 28), lesdites surfaces de sortie (10, 28) sont pressées l'une contre l'autre, en grande mesure sans tolérance, par précontrainte, et sachant qu'une hauteur de fente est réglée par des écarteurs (25, 32) entre les surfaces de sortie (10, 28), **caractérisé en ce que** l'une (10) des surfaces de sortie est formée dans le corps de masque (1) et que l'autre surface de sortie (28) complémentaire est formée, avec un dispositif de fermeture (20, 26), sur une bague de sécurité (31) au moyen de laquelle a lieu une fixation du corps de masque (1) et de la pièce de raccordement (3, 18) l'un par rapport à l'autre.

2. Masque respiratoire selon la revendication 1,
**caractérisé en ce que** l'exemption de tolérance est réalisée par le contact de composants (1, 31) sur trois points au plus.

3. Masque respiratoire selon revendication 1 ou 2,
**caractérisé en ce que** l'exemption de tolérance est réalisée par un contact des composants (1, 31) le long de deux lignes de guidage (33, 34).

4. Masque respiratoire selon l'une des revendications précédentes,
**caractérisé en ce que** les composants (1, 31) sont contraints élastiquement l'un par rapport à l'autre pour former la fente d'expiration (14) au moins prévue.

5. Masque respiratoire selon l'une des revendications précédentes
**caractérisé en ce que** la fente d'expiration (14) au moins prévue est disposée sur le corps de masque (1), dans la région d'un raccord (13), qui reçoit la pièce de raccordement articulée (3, 18).

6. Masque respiratoire selon la revendication 5,
**caractérisé en ce que** le raccord (13) est une bague de centrage (13) pour la mise en place de la bague de sécurité (31), et q u e la fente d'expiration (14) au moins prévue est disposée dans la zone formée entre la surface de sortie (10) et la bague de centrage (13).

7. Masque respiratoire selon l'une des revendications précédentes,
**caractérisé en ce que** la pièce de raccordement (3, 18) articulée est réalisée sous la forme d'un joint à rotule.

8. Masque respiratoire selon les revendications 6 ou 6 et 7,
**caractérisé en ce que** la bague de sécurité (31) est positionnée, en commun avec la pièce de raccordement (3, 18), dans la région de la bague de centrage (13) du corps de masque (1).

9. Masque respiratoire selon revendication 7 ou 8,
**caractérisé en ce que**, dans la région située entre le joint à rotule (18) et la bague de sécurité (31), maintenue sur le corps de masque (1), s'effectue une tension élastique du joint à rotule (18) à l'intérieur de la cage de roulement (24).

10. Masque respiratoire selon la revendication 9,
**caractérisé en ce que** la fixation du joint à rotule (18) est effectuée dans une cage de roulement (24), par la bague de centrage (13), qui, dans la cage de roulement (24), porte, au moins par sections, à l'extérieur.
